# EUROPEAN PATENT SPECIFICATION

(11) **EP 0 594 630 B1**
(45) Date of publication and mention of the grant of the patent: **15.11.1995**
(21) Application number: 92907149.6
(22) Date of filing: 23.03.1992
(51) Int. Cl.: G01N 33/00, G01N 27/28

(54) **SENSOR UNIT FOR USE IN HAZARDOUS AREAS AND TOOL FOR DISMANTLING AND REASSEMBLING SAME**
SENSOREINHEIT ZUR VERWENDUNG INNERHALB GEFÄRLICHER RÄUME UND DERSELBEN WERKZEUG ZUM MONTIEREN UND DEMONTIEREN
UNITE DE DETECTION POUR ZONES DE DANGER ET OUTIL DE DEMONTAGE ET DE REMONTAGE DE CELLE-CI

(30) Priority: 17.07.1991 GB 91154286
(43) Date of publication of application: 04.05.1994
(73) Proprietor: GROVELEY DETECTION LIMITED, Christchurch, Dorset BH23 3HB (GB); GIBBON, Cedric Stanley, Sanderstead, Surrey CR2 9EF (GB)
(72) Inventor: GIBBON, Cedric, Stanley, Sanderstead, Surrey CR2 9EF (GB); BENNET, Robert, John, Christchurch, Dorset BH23 2RD (GB)
(74) Representative: Lucas, Brian Ronald
(86) International application number: EP9200649
(87) International publication number: WO9302355

(56) References cited:
- WO-A-84/04967
- DE-U- 9 100 207
- GB-A- 2 053 492
- GB-A- 2 067 294
- US-A- 3 278 408
- US-A- 4 352 099

## Description

This invention relates to sensor units for use in hazardous areas and, more particularly but not exclusively, is concerned with a gas sensor unit. The invention also relates to a tool for dismantling and reassembling said sensor units.

Gas sensor units are strategically placed at many locations on oil platforms. Typically, a large production platform in the North Sea will have between 400 and 600 gas sensor units, each designed to actuate an alarm in the event that the ambient air contains more than a predetermined amount of methane, other combustible vapour or toxic gases.

The gas sensor units presently in common use comprise a gas detector unit mounted in a unitary housing. The entire gas sensor unit often needs replacement every two to three months. This is a time-consuming and expensive exercise. In particular, many gas sensor units are, for various reasons, mounted more than 2.0m above local deck level. In order to replace such gas sensor units a scaffolding has first to be erected to enable an electrician to work on the small connecting screws. The electrician then has to obtain a "hot working" permit to enable him to work on a junction box adjacent the gas sensor unit. The supply of power to the junction box is disconnected and the junction box is opened. The wires connected to the gas sensor units are then disconnected and the gas sensor unit and its connecting wires are removed as a complete unit which is then replaced. After replacing the gas sensor unit and reconnecting the connecting wires the junction box is resealed, the supply of power reconnected and the gas sensor unit calibrated. The scaffolding is then dismantled. This entire operation typically takes approximately 11 man hours. Similar problems also occur when other sensor units have to be replaced, for example temperature and pressure sensor units.

US-A-4 352 099 and WO-A-84 04967 both disclose a sensor unit for use in hazardous areas, said sensor unit comprising a housing having a first part and a second part removably mounted on said first part, a base mounted on said first part and a detector component which is plugged into said base.

In order to change the detector component the sensor unit must first be electrically isolated. The second part is then removed to expose the detector component which can be withdrawn and replaced. It will be appreciated that scaffolding must be erected to gain access to most sensor units. Furthermore, whilst replacing the detector component is relatively easy on a fine summer's day, manipulating a delicate sensor unit in the inclement conditions which can exist on North Sea oil platforms can be impossible.

One aspect of the present invention aims, at least in its preferred embodiments, to simplify the entire procedure, and is characterized in that said detector component is mounted on said second part so that when said second part is separated from said first part said detector component is withdrawn from said base.

Preferably, said first part includes a threaded tongue for mounting said sensor unit on a junction box.

Advantageously, said second part comprises an inner member and a locking ring rotatable with respect to said inner member.

Preferably, said sensor unit includes a circlip to inhibit separation of said inner member and said locking ring.

Advantageously, said sensor unit includes a locking member disposed between said inner member and said locking ring, rotation of said locking ring relative to said inner member in one sense being effective to urge said locking member radially outwardly.

Preferably, said sensor unit includes a locating pin for aligning said first part and said inner member in a predetermined orientation.

Advantageously, said sensor unit includes a locating pin holding said base in a predetermined orientation with respect to said first part.

Preferably, said base is provided with sockets for receiving pins on said detector component.

Advantageously, said detector component is slideably mounted in said inner member.

Preferably, said detector component is designed for detecting gas.

In one embodiment, said second part is provided with flats for remote rotation by a tool.

Preferably, said flats are let into recesses so that said tool can be manipulated to rotate said second part and withdraw it from or inset it in said first part.

Advantageously, said second part is provided with at least one keyway for receiving a tool for rotating said second part.

Advantageously, said second part is provided with two keyways disposed on opposite sides of said second part.

Preferably, an annular groove is formed between said first part and said second part when said first part and said second part are connected.

Advantageously, said keyways open into said annular groove.

Preferably, said first part is provided with an annular groove for supporting a tool during remote assembly of said sensor unit.

The present invention also provides a tool for dismantling and reassembling a sensor unit characterized in that said tool comprises a cup for accommodating said second part of the said gas sensor and a first lever which is pivotally mounted on said cup and which has a first tooth which, when said second part enters said cup enters said keyway on said second part and constrains said second part for rotation with said tool.

Preferably, said tool comprises a second lever pivotally mounted on said cup opposite said first lever, each lever having a first tooth which, when said second part enters said cup enters a respective keyway on said second part and constrains said second part for rotation with said tool.

Advantageously, said tool includes means for biasing the forward ends of said levers towards one another.

Preferably, said cup is provided with a longitudinally extending slot associated with each lever for allowing the first tooth on said lever to pass through said cup.

Advantageously, when said first part of said sensor unit is provided with a groove for supporting said tool during remote assembly of said sensor unit, at least one lever is provided with a second tooth longitudinally spaced from said first tooth so that when said cup is mounted on said sensor unit said first tooth can enter said annular groove on said first part to support said tool and said second tooth can enter said keyway on said second part of said housing.

Preferably, said tool includes a rod having an end cap, and a spring disposed between said end cap and said cup whereby said cup can be moved towards and away from said end cap.

Advantageously, the or one of said cup and the or one of said levers is provided with a pin and the other is provided with a cam surface and a groove, the arrangement being such that when said end cap is moved towards said cup said pin moves along said cam surface causing the forward end of the or each lever to move away from said cup; sufficient relative movement of said end cap towards said cup causing said pin to enter said groove and lock said lever against return movement.

Preferably, means are provided to inhibit movement of said end cap towards said cup until sufficient torque is applied to said rod.

Advantageously, said end cap comprises a part of an end cap assembly comprising said end cap and a driver portion, and said means comprises a resilient member arranged to resiliently resist turning movement between said end cap and said driver portion.

Preferably, said means further comprises a bayonet slot in said driver portion, a longitudinally extending slot in said end cap, and a carrier attached to said cup and having a rod extending through said longitudinally extending slot and said bayonet slot.

Advantageously, said tool includes a spacer disposable in said cup to facilitate insertion of said first tooth in said keyway during a dismantling operation.

Preferably, said tool includes a rod for positioning said tool.

Advantageously, said tool includes a tube for conveying calibration gas from the end of said rod remote from said cup to said cup.

Preferably, said rod is hollow and said tube extends internally thereof.

Advantageously, said tool includes a spacer having a chamber for receiving calibration gas, a flange surrounding said chamber for engagement with said second part of said sensor unit and a bleed channel for allowing calibration gas introduced into said tube remote from said cup to displace air from said tube and said chamber.

Preferably, said tool includes a cylinder of calibration gas connected to said tube.

Advantageously, a valve is mounted on said rod for controlling the flow of calibration gas through said tube.

Preferably, said tool includes a cylinder of calibration gas mounted on said rod remote from said cup.

For a better understanding of the present invention, reference will now be made, by way of example, to the accompanying drawings, in which:-
Figure 1 is a cross-section of one embodiment of a sensor unit in accordance with the present invention mounted in its operative position;
Figure 2 is a cross-section of a second embodiment of a sensor unit in accordance with the present invention mounted in its operative position;
Figure 3 is a side elevation of a tool in accordance with the present invention ready to dismantle the sensor unit shown in Figure 2;
Figure 4 is a cross-section of the tool shown in Figure 3 in its release position;
Figure 5 is a top plan view of the tool shown in Figure 3;
Figure 6 is a section on line VI-VI of Figure 4; and
Figure 7 is a cross-sectional view of a spacer for use during calibration.

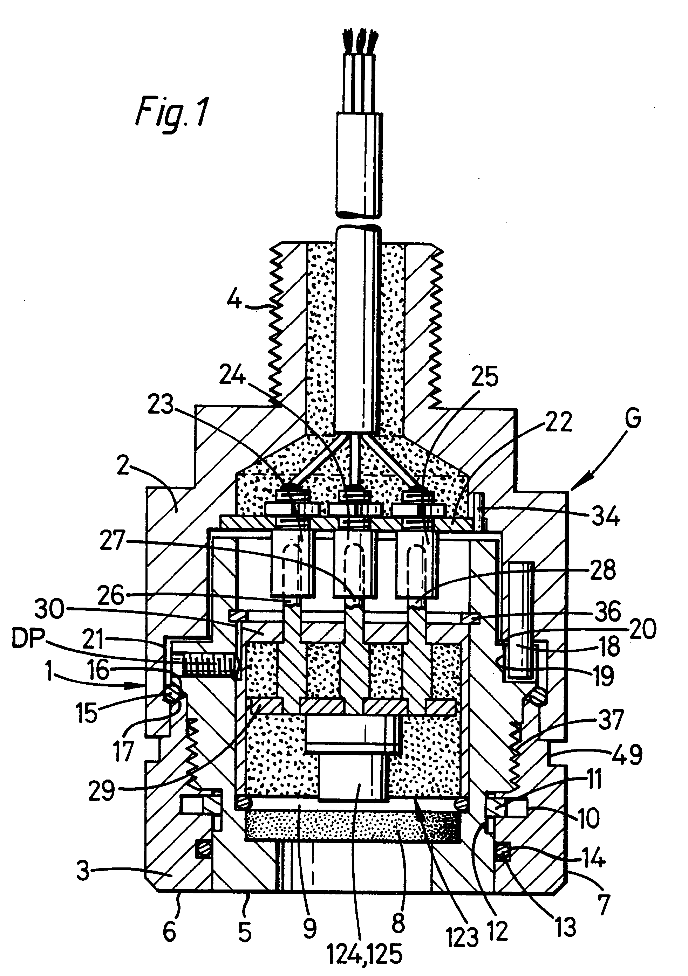

Referring to Figure 1 of the drawings there is shown a gas sensor unit G which comprises a housing 1. The housing 1 comprises a first part 2 and a second part 3. The first part 1 is provided with a threaded tongue 4 which can be securely attached to a female fitting on a flame proof junction box mounted on the superstructure of an oil platform (not shown). Secure attachment may be achieved by, for example a pair of locknuts and the use of a setting compound on the threads.

The second part 3 comprises an inner member 5 and a locking ring 6 which are connected by threads 37. The locking ring 6 is disposed circumjacent the inner member 5 and is provided with a plurality of recessed flats or slots 7 to facilitate gripping and rotation of said locking ring 6 with a tool.

A porous sintered member 8 is mounted in one end of said inner member 5 and allows the passage of ambient air to the inside 9 of the housing 1.

The locking ring 6 is provided with an internal grove 10 which houses a circlip 11 which extends radially into an annular groove 12 cut in the periphery of the inner member 5. This arrangement enables the locking ring 6 to move axially relative to the inner member 5 by an amount determined by engagement of the circlip 11 with the sides of the annular groove 12.

A seal 13 is provided in an annular groove 14 in the locking ring 6 as shown. The seal 13 may be of plastics material and/or metal as desired.

A locking member 15, which may be of plastics material and/or metal is disposed between an abutment 16 on the inner member 5 and the inner end 17 of the locking ring 6. It will be noted that the inner end 17 of the locking ring 6 and the abutment 16 on the inner member 5 both slope inwardly so that when said locking ring 6 and said inner member 5 are urged together the locking member 15 is urged radially outwardly. If the locking member 15 is of resilient material then it conveniently comprises an "O-ring" seal. However, if the locking member 15 is of non-resilient material then it conveniently takes the shape of a circlip subtending approximately 350°.

A locating pin 18 is mounted in the first part 2 and projects forwardly to enter a notch 19 cut in a face 20 of the inner member 5.

In order to mount the second part 3 on the first part 2 the locking ring 6 is first rotated relative to the inner member 5 so that the abutment 16 and inner end 17 move apart thereby allowing locking member 15 to move radially inwardly.

The second part 3 is then inserted in the first part 2 and rotated until the locating pin 18 enters the notch 19. The second part 3 is then pressed fully inwardly. The locating pin 18 holds the inner member 5 against rotation so that when the locking ring 6 is rotated the inner end 17 of the locking ring 6 moves axially towards the abutment 16 and urges the locking member 15 radially outwardly into an undercut portion 21 of the first part 2 thereby inhibiting disengagement of said first part 2 and said second part 3.

A base 22 having three sockets 23, 24, 25 is set in the first part 2 and is permanently wired to connectors in the junction box (not shown) on which the first part 2 is securely mounted. The position of the base 22 with respect to the first part 2 is determined by a locating pin 34.

A gas detector 123 which comprises a pair of pellistors 124, 125 and a ballast resistor (not shown) is mounted on a separate board 29 having three pins 26, 27 and 28 which engage sockets 23, 24 and 25 respectively. The board 29 is potted in a cup 30 which is located relative to the inner member 5 by a dog point grab screw DP which passes through a hole in the inner member 5 and into an axially extending slot in the cup 30. The cup 30 is retained axially in the inner member 5 by a circlip 36.

The pins 26, 27 and 28 are of a length such that they do not enter the sockets 23, 24, 25 until the locating pin 18 enters the notch 19. As a result damage to the pins 26, 27 and 28 is inhibited.

In order to change the gas detector 123 the electrician simply rotates locking ring 6 to release the locking member 15. The whole second part 3, complete with board 29, can then be withdrawn in one piece. A replacement second part 3, complete with a new gas detector 123, can then be offered up to the first part 2 and inserted. The second part 3 is then rotated under light axial pressure until the locating pin 18 aligns with the notch 19. As the second part 3 is pushed home the pins 26, 27 and 28 enter and engage the sockets 23, 24 and 25.

It will be noted that by the time the pins 26, 27 and 28 first contact the sockets 23, 24 and 25 the locking member 15 is already in contact with the first part 2 of the housing 1. Furthermore, since the voltage and current involved are very small (typically 3-32v and up to 350 ma) the chances of a spark sufficient to start a fire are minimal. In any event good practice would be to electrically isolate the gas sensor unit G at least five minutes prior to replacing a gas detector 123.

Once the second part 3 is fully home the locking ring 6 is simply rotated to expand the locking member 15 into its locking position.

It will be appreciated that even though the gas detector 123 is relatively delicate the construction of the second part 3 makes it possible for the gas detector 123 to be replaced without easy access to the gas sensor unit G. The gas detector 123 can readily be replaced in units up to 2m from the ground. For higher units, up to 3m, a tool resembling a long spanner can be used to loosen and tighten the locking ring 6.

In order to facilitate axial insertion and removal of the second part 3 the flats 7 on the locking ring 6 may be recessed so that axial movement of the tool is transferred to the locking ring 6. The tool itself can be made of a strong lightweight plastics material to enable the electrician to manoeuvre the second part without difficulty.

Various modifications to the arrangement can be described are envisaged, for example the gas detector could simply be plugged into the base as a separate and distinct operation from mounting the second part on the first part.

If desired the gas detector could be plugged into the base via a bayonet or other connector although the push fit connection described is very much preferred.

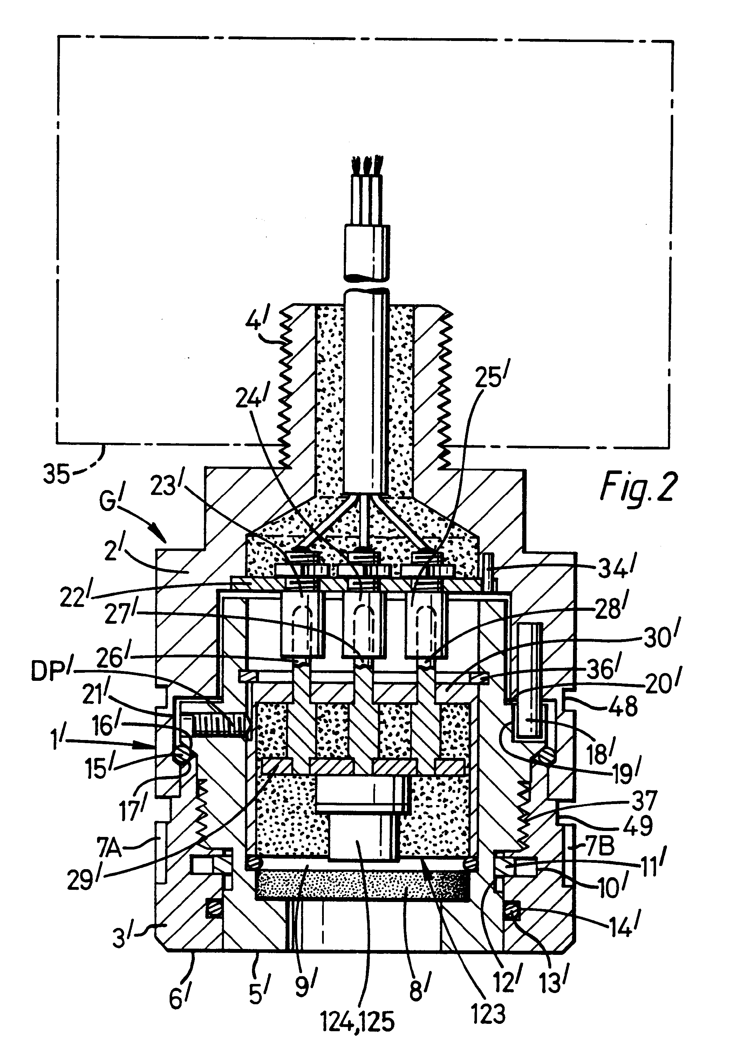

Figure 2 shows a gas sensor unit G′ which is generally similar to the gas sensor unit G but which has been modified so that the gas detector 123 can be replaced remotely, for example at distances up to 7.8m from the electrician.

Those parts of the gas sensor unit G′ which correspond with parts in the gas sensor G have been identified by the same reference numeral but with the addition of an apostrophe.

The differences between the gas sensor unit G′ and the gas sensor G are that:-
1. The first part 2′ of the gas sensor unit G′ is provided with an annular groove 48; and
2. The slots 7 have been replaced by two keyways 7A, 7B.

In order to facilitate remote replacement of the gas detector 123 use is made of the annular groove 48 and the keyways 7A and 7B.

Before describing the tool in greater detail it should be borne in mind that gas sensor units are conventionally mounted as shown in Figure 2, i.e., to a flame proof junction box 35 with the porous sintered member 8 facing downwardly.

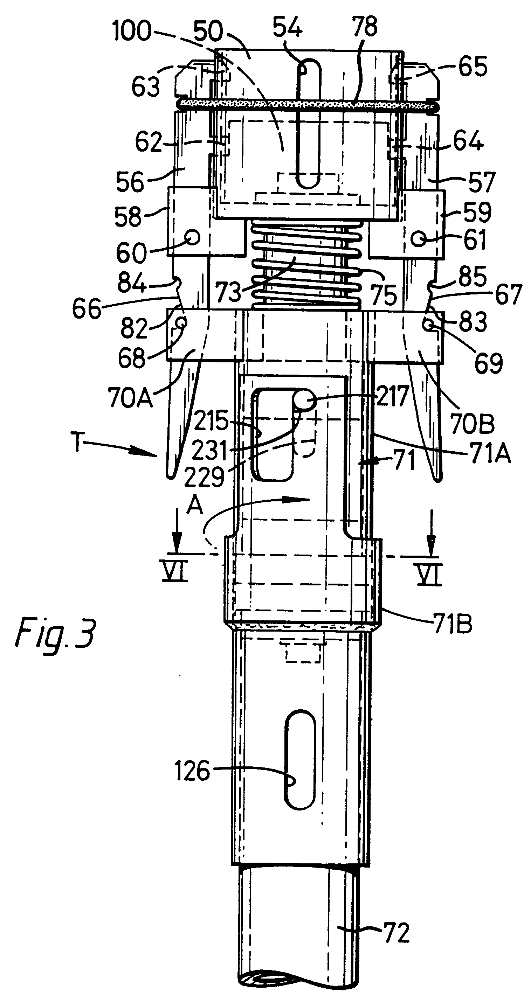

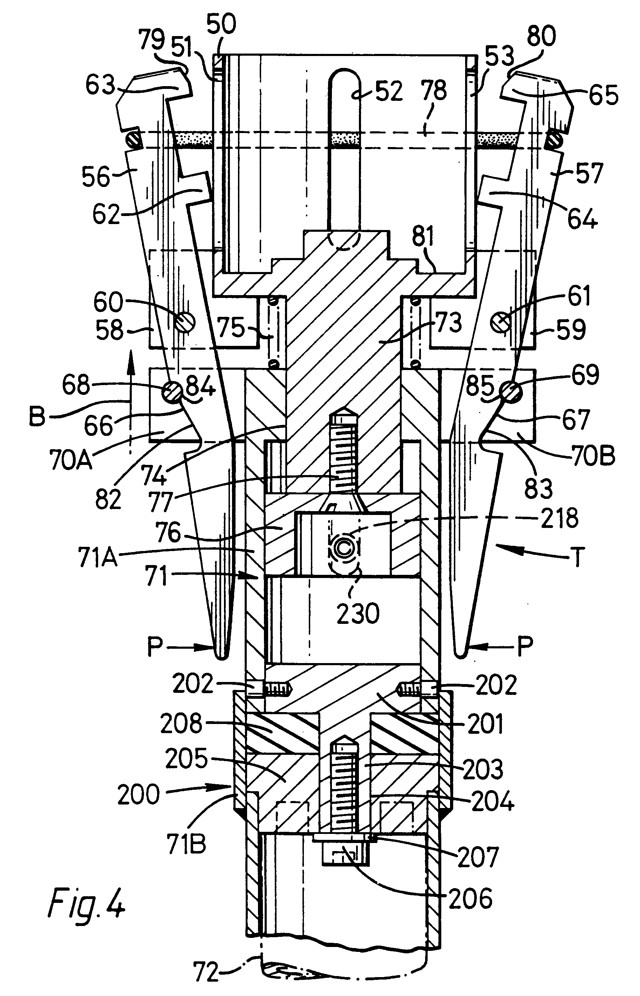

Turning now to Figures 3 and 4, there is shown a tool 'T' for dismantling the gas sensor unit G′. The tool 'T' comprises a cup 50 which is provided with four longitudinally extending slots 51, 52, 53 and 54. Slots 51 and 53 are dispose opposite one another.

Two levers 56, 57 are pivotally mounted on bifurcated ears 58, 59 which project outwardly on opposite sides of the cup 50. The lever 56 is pivoted on pin 60 whilst lever 57 is pivoted on pin 61.

The forward end of lever 56 is provided with two teeth 62, 63 which face teeth 64, 65 on the forward end of lever 57.

The rearward end of lever 56 is provided with a cam surface 66 whilst the rearward end of lever 57 is provided with a cam surface 67.

The cam surfaces 66 and 67 co-operate with pins 68, 69 which extend between bifurcated ears 70A, 70B.

The bifurcated ears 70A, 70B form part of an end cap 71A of an end cap assembly 71 which is mounted on a hollow multi-section fibreglass rod 72 which may be extended section by section to the desired length.

The cup 50 has a stem portion 73 which extends slidably downwardly through a hole 74 in the end cap 71A. The cup 50 is biased upwardly by a spring 75 which acts between the bottom of the cup 50 and the top of the end cap 71A.

Upward movement of the cup 50 is limited by a carrier 76 (Figure 4).

The forward portions of the levers 56, 57 are biased together by an elastic band 78.

The end cap assembly 71 comprises the end cap 71A and a driver portion 71B which are interconnected by a torque assembly 200 (Figure 4).

The torque assembly 200 comprises an upper member 201 which is secured to the bottom of the end cap 71A by a plurality of socket screws 202. The upper member 201 has a downwardly extending portion 203 which is pivotally mounted in a bore 204 in a block 205 fast with the driver portion 71B. Axial separation of the end cap 71A from the driver portion 71B is inhibited by a bolt 206 which is threadedly connected to the downwardly extending portion 203 and a washer 207.

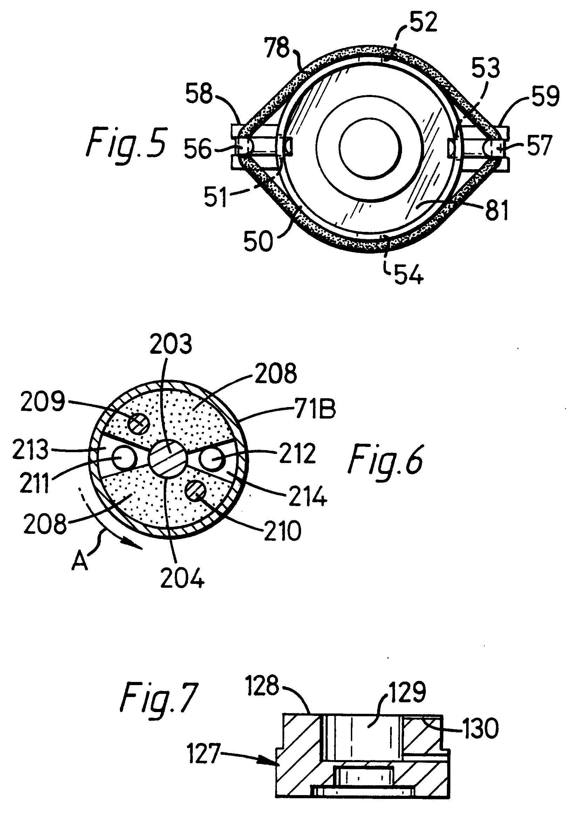

A resilient bush 208 is disposed between the upper member 201 and the block 205. As can be seen in Figure 6, two studs 209, 210 extend downwardly from the upper member 201 into the resilient bush 208. Similarly, two studs 211, 212 project upwardly from the block 205 into gaps 213, 214 in the resilient bush 208.

The overall effect of the torque assembly 200 is such that the end cap 71A can be turned relative to the driver portion 71B against the restoring force of the resilient bush 208.

The extend to which the end cap 71A can turn relative to the driver portion 71B is determined by two bayonet slots 215, 216 which are cut in upstands which extend upwardly on opposite sides of the driver portion 71B. In particular, two rods 217, 218 are mounted on opposite sides of the carrier 76 and project radially outwardly through longitudinally extending slots 229, 230 in the end cap 71A and through the bayonet slots 215, 216 respectively.

In the rest position shown in Figure 3 the cup 50 is inhibited from moving towards the end cap 71A by the rod 217 engaging the horizontal edge 231 of the bayonet slot 215.

If the driver portion 71B is rotated by the rod 72 relative to the end cap 71A in the direction of arrow "A" (Figure 3) the rod 217 (and rod 218) will move into the elongate portions of their respective bayonet slots 215, 216 thereby allowing cup 50 to be moved towards end cap 71A for a reason which will be explained later.

Before dismantling gas sensor G′, a spacer 100 is placed inside the cup 50. The perimeter of the upper portion of the spacer 100 is cut away so that the elastic band 78 biases the forward ends of the levers 56, 57 radially inwardly until the teeth 62, 63, 64, 65 project through the longitudinally extending slots 51, 53.

After ensuring that the gas sensor unit G′ to be dismantled has been electrically isolated and a sufficient time has elapsed for the safe discharge of any residual electricity the cup 50 is then raised towards the gas sensor unit G′, sections being added to the rod 72 as required.

As the cup 50 is pushed up over the second part 3′ of the housing chamfers 79, 80 on the teeth 63, 65 in conjunction with the chamfer on the bottom of the locking ring 6′ deflect the levers 56, 57 outwardly and permit cup 50 to be pushed upwardly until the bottom 81 of the locking ring 6 engages the top of the spacer 100. At this point the teeth 63, 65 are just below the annular groove 49 between the first part 2′ and the second part 3′.

The rod 72 is then rotated until the teeth 63, 65 align with the keyways 7A and 7B which they drop into under the influence of the elastic band 78.

Once the teeth 62, 63 are in the keyways 7A, 7B the rod 72 is rotated anti-clockwise (approximately 1.5 turns) to allow the locking member 15 to move radially inwardly. At this point the second part 3′ can be pulled downwardly from the first part 2′ together with the gas detector 123. It will be noted that as the rod 72 is rotated anti-clockwise torque is transmitted from the driver portion 71B to the end cap 71A via the rods 217, 218.

The second part 3′ is held within the cup 50 by the teeth 63, 65 during the separating and lowering operation. When the cup 50 reached ground level the rearward ends of the levers 56, 57 are pressed together in the direction of arrows P to release the second part 3′. The insert 100 is also removed from the cup 50 in preparation for the reassembly operation.

At ground level a replacement second part 3′ containing a new gas detector 123 is placed in the cup 50. It should be noted that the gas detector 123 could be replaced during servicing. However, it is more convenient to have pre-prepared units to hand.

The replacement second part 3′ is inserted into the cup 50 until the bottom of the second part 3′ engages the bottom 81 of the cup 50. It is then rotated until the teeth 62 and 64 enter the keyways 7A, 7B and lock the second part 3′ in position.

The second part 3′ is then raised until the top of the cup 50 is circumjacent the first part 2′ of the sensor unit.

The second part 3′ is then pressed upwardly into the first part 2′. As the second part 3′ enters the cup 50 the chamfers 79, 80 cause the forward ends of the levers 56, 57 to move apart. This action partially withdraws teeth 62, 64 from the keyways 7A, 7B. However, sufficient of the teeth 62, 64 still project into the keyways 7A, 7B to ensure that rotation of the rod 72 is applied to the second part 3′.

The rod 72 is simultaneously gently urged upwardly and rotated until the locating pin 18 enters the notch 19. Location of the locating pin 18 in the notch 19 can be easily felt in the rod 72.

As the second part 3′ moves fully home the teeth 63, 65 enter the annular groove 48 and support the rod 72. Rod 72 is then rotated clockwise to move the locking member 15′ into its operative position (approximately 1.5 turns).

It will be noted that if the second part 3′ is not pressed fully home the teeth 63, 65 will not enter the annular groove 48 and this will result in the second part 3′ being withdrawn if the rod 72 is tugged downwardly. In this connection it is of extreme importance that the second part 3′ is properly mounted on the first part 2′ to ensure the flame proof integrity of the housing 1′.

It will be recalled that once the second part 3′ is in its final position the teeth 63, 65 are in the annular slot 48 supporting the rod 72.

In order to release the tool 'T', rod 72 is turned in the direction of arrow 'A' against the resistance of resilient bush 208 until rods 217, 218 lie over the axially extending portions of their respective bayonet slots 215, 216. This ensures that the second part 3′ is correctly tightened. Whilst applying the necessary torque to the rod 72, the rod 72 is pushed firmly upwards to compress spring 75. This action allows the driver portion 71B to move upwardly relative to the end cap 71A. This, in turn, causes pins 68, 69 to travel along the sloping portions 82, 83 of the cam surfaces 66, 67 and to enter grooves 84, 85 under the influence of the elastic band 78.

As the pins 68, 69 travel along the sloping portions 82, 83 the forward ends of the levers 56, 57 move apart to withdraw the teeth 63, 65; 62, 64 from the annular groove 48, 49 and the keyways 7A, 7B respectively. Once the pins 68, 69 have entered the grooves 84, 85 the levers 56, 57 are permanently held in position (Figure 4) until the tool is reset. The rod 72 can then be lowered.

After the tool 'T' has been lowered the rearward ends of the levers 56, 57 are pressed together in the direction of arrows P and this releases pins 68, 69 from the grooves 84, 85. The spring 75 then moves the cup 50 away from the rod 72 and resets the tool 'T' to its initial position.

It will be appreciated that safety is paramount in the environments in which the subject gas sensors are used. Accordingly, after the electrician has finished the supervisor may check his work by inserting the spacer 100 as shown in Figure 3, causing the teeth 63, 65 to engage in the keyways 7A, 7B as in a dismantling operation and tugging downward on the rod 72. The tool 'T' can be released by rotating the rod 72 in the direction of arrow 'A' until sufficient torque is applied to bring the rods 217, 218 into alignment with the longitudinal parts of the bayonet slots 215, 216 and pushing the rod 72 upwardly to lock the levers 56, 57 in their open position.

It will be appreciated that, in most cases, the need to erect and dismantle scaffolding has been obviated. The need to open and reseal the junction box (a flameproof enclosure) has been obviated. The need to manipulate tiny connecting screws in conditions which can be as hostile as any in the world has been obviated. As a result the time can be reduced to a matter of minutes to replace the unit. However, the gas detector 123 still has to be calibrated.

At the present time calibration is carried out by first allowing the gas detector to reach operating temperature. This typically takes about one hour. A quantity of gas of a known composition is then admitted to the gas detector via the porous sintered member 8 and the response noted at a remote control centre.

In order to facilitate this operation a spacer 127 (Figure 7) is placed in the cup 50 of the tool. A cylinder of calibration gas (not shown) is attached to the bottom of the rod 72 and a plastic tube is used to connect the cylinder of calibration gas to the spacer 127 via a hand valve mounted on the rod 72 or on the cylinder. The plastic tube (not shown) passes through the centre of the rod 72, emerges through the slot 126, enters the cup 50 through longitudinally extending slot 52 and opens into a chamber 129 in the spacer 127. The spacer 127 is similar to the spacer 100 with the exception that it has an annular flange 128 adapted to press against the bottom of the locking ring 6. A small bleed channel 130 is cut in the upper surface of the annular flange 128.

In use, the tool 'T' is first suspended from the gas sensor unit G′ by engaging the teeth 63, 65 in the annular groove 49. The hand valve is then opened to admit calibration gas to the chamber 129. As the calibration gas flows into the chamber 129 it displaces air to atmosphere via the bleed channel 130. After a period of a few minutes the composition of the gas in the chamber 129 is substantially identical to the composition of the calibration gas and calibration can proceed.

At the end of the calibration procedure the rod 72 is removed as described with reference to the assembly procedure, i.e., by twisting the rod 72, raising the rod 72 until the levers 56, 57 are locked apart and then lowering the rod 72.

Various modifications to the procedure described are envisaged. For example, it might be possible to dispense with the spacer 100 for dismantling the gas sensor unit G′ although this is not recommended.

In addition, the torque assembly 200 might be dispensed with. However, as this helps ensure that the tool 'T' is not removed before the second part 3′ is fully mounted on the first part 2′ this omission is also not recommended.

## Claims

1. A sensor unit (G; G′) for use in hazardous areas, said sensor unit comprising a housing (1; 1′) having a first part (2; 2′) and a second part (3; 3′) removably mounted on said first part (2; 2′), a base (22; 22′) mounted on said first part (2; 2′), and a detector component (123; 123′) which is plugged into said base (22; 22′), characterized in that said detector component (123; 123′) is mounted on said second part (3; 3′) so that when said second part (3; 3′) is separated from said first part (2; 2′) said detector component (123; 123′) is withdrawn from said base (22; 22′).

2. A sensor unit as claimed in Claim 1, characterized in that said first part (2; 2′)includes a threaded tongue (4, 4′) for mounting said sensor unit (G, G′) on a junction box (35).

3. A sensor unit as claimed in Claim 1 or 2, characterized in that said second part (3; 3′) comprises an inner member (5; 5′) and a locking ring (6; 6′) rotatable with respect to said inner member (5; 5′).

4. A sensor unit as claimed in Claim 3, characterized in that it includes a circlip (11; 11′) to inhibit separation of said inner member (5; 5′) and said locking ring (6; 6′).

5. A sensor unit as claimed in Claim 3 or 4, characterized in that it includes a locking member (15; 15′) disposed between said inner member (5; 5′) and said locking ring (6; 6′), rotation of said locking ring (6; 6′) relative to said inner member (5; 5′) in one sense being effective to urge said locking member (15; 15′) radially outwardly.

6. A sensor unit as claimed in Claim 3, 4 or 5, characterized in that it includes a locating pin (18; 18′) for aligning said first part (2; 2′) and said inner member (5; 5′) in a predetermined orientation.

7. A sensor unit as claimed in any preceding Claim, characterized in that it includes a locating pin (34; 34′) holding said base (22; 22′) in a predetermined orientation with respect to said first part (2; 2′).

8. A sensor unit as claimed in any preceding Claim, characterized in that said base (22; 22′) is provided with sockets (23, 24, 25; 23′, 24′, 25′) for receiving pins (26, 27, 28; 26′, 27′, 28′) on said detector component (123; 123′).

9. A sensor unit as claimed in Claim 3, 4, 5 or 6, or Claim 7 or 8 when dependent on Claim 3, characterized in that said detector component (123; 123′) is slideably mounted in said inner member (5; 5′).

10. A sensor unit as claimed in any preceding Claim, characterized in that said detector component (123; 123′) is designed for detecting gas.

11. A sensor unit as claimed in any preceding Claim, characterized in that said second part (3; 3′) is provided with flats (7) for remote rotation by a tool.

12. A sensor unit as claimed in Claim 11, characterized in that said flats (7) are let into recesses so that said tool can be manipulated to rotate said second part and withdraw it from or insert it in said first part.

13. A sensor unit as claimed in any preceding Claim, characterized in that said second part (3) is provided with at least one keyway (7A, 7B) for receiving a tool ('T') for rotating said second part (3′).

14. A sensor unit as claimed in Claim 13, characterized in that said second part (3′) comprises two keyways (7A, 7B) disposed on opposite sides of said second part (3′)

15. A sensor unit as claimed in Claim 13 or 14, characterized in that an annular groove (49) is formed between said first part (2′) and said second part (3′) when said first part (2′) and said second part (3′) are connected.

16. A sensor unit as claimed in Claim 14 and 15, characterized in that said keyways (7A, 7B) open into said annular groove (49).

17. A sensor unit as claimed in Claim 13, 14, 15 or 16, characterized in that said first part (2′) is provided with an annular groove (48) for supporting a tool ('T') during remote assembly of said sensor unit (G′).

18. A tool for dismantling and reassembling a sensor unit as claimed in Claim 13, characterized in that said tool ('T') comprises a cup (50) for accommodating said second part (3) of said gas sensor (G′), and a first lever (56, 57) which is pivotally mounted on said cup (50) and which has a first tooth (63, 65) which, when said second part (3′) enters said cup (50) enters said keyway (7A, 7B) on said second part (3′) and constrains said second part (3′) for rotation with said tool ('T').

## Patentansprüche

1. Sensoreinheit (G; G′) zur Verwendung in gefährlichen Räumen, bestehend aus einem Gehäuse (1; 1′) mit einem ersten Teil (2; 2′) und einem zweiten Teil (3; 3′), abnehmbar an dem ersten Teil (2; 2′) befestigt, einer Grundplatte (22; 22′), befestigt an dem ersten Teil (2; 2′) und einem Detektorteil (123; 123′), das in die Grundplatte (22; 22′) gesteckt ist, dadurch gekennzeichnet, daß der Detektorteil (123; 123′) an dem zweiten Teil (3; 3′) so befestigt ist, daß, wenn der zweite Teil (3; 3′) von dem ersten Teil getrennt wird, der Detektorteil (123; 123′) von der Grundplatte (22; 22′) abgezogen wird.

2. Sensoreinheit nach Anspruch 1, dadurch gekennzeichnet, daß der erste Teil (2; 2′) einen mit einem Gewinde versehenen Ansatz (4; 4′) zur Befestigung der Sensoreinheit (G; G′) an einem Anschlußkasten (35) enthält.

3. Sensoreinheit nach Anspruch 1 oder 2, dadurch gekennzeichnet, daß der zweite Teil (3; 3′) aus einem Innenteil (5; 5′) und einem Sicherungsring (6; 6′) besteht, der gegen das Innenteil (5; 5′) verdrehbar ist.

4. Sensoreinheit nach Anspruch 3, dadurch gekennzeichnet, daß er einen Seegerring (11; 11′) enthält, um die Trennung dessen Innenteils (5; 5′) und des Sicherungsrings (6; 6′) zu verhindern.

5. Sensoreinheit nach Anspruch 3 oder 4, dadurch gekennzeichnet, daß sie ein Sicherungsteil (15; 15′), angeordnet zwischen dem Innenteil (5; 5′) und dem Sicherungsring (6; 6′), aufweist und die Drehung des Sicherungsrings (6; 6′) relativ zum Innenteil (5; 5′) in eine Richtung bewirkt, daß das Sicherungsteil (15; 15′) radial auswärts gedrängt wird.

6. Sensoreinheit nach einem der Ansprüche 3, 4 oder 5, dadurch gekennzeichnet, daß sie einen Sicherungsstift (18; 18′) zum Ausrichten des ersten Teils (2; 2′) und des Innenteils (5; 5′) in eine vorbestimmte Richtung aufweist.

7. Sensoreinheit nach einem der vorhergehenden Ansprüche, dadurch gekennzeichnet, daß sie einen Sicherungsstift (34; 34′) zum Halten der Grundplatte (22; 22′) in einer vorbestimmten Ausrichtung zum ersten Teil (2; 2′) aufweist.

8. Sensoreinheit nach einem der vorhergehenden Ansprüche, dadurch gekennzeichnet, daß die Grundplatte (22; 22′) mit Buchsen (23, 24, 25; 23′, 24′, 25′) zur Aufnahme von Stiften (26, 27, 28; 26′, 27′, 28′) am Detektorteil (123; 123′) versehen ist.

9. Sensoreinheit nach einem der Ansprüche 3, 4, 5 oder 6 oder Anspruch 7 oder 8, wenn sie vom Anspruch 3 abhängig sind, dadurch gekennzeichnet, daß der Detektorteil (123; 123′) verschiebbar an dem Innenteil (5; 5′) montiert ist.

10. Sensoreinheit nach einem der vorhergehenden Ansprüche, dadurch gekennzeichnet, daß der Detektorteil (123; 123′) für das Aufspüren von Gas bestimmt ist.

11. Sensoreinheit nach einem der vorhergehenden Ansprüche, dadurch gekennzeichnet, daß der zweite Teil (3; 3′) mit Ansatzflächen (7) für eine Ferndrehung durch ein Werkzeug versehen ist.

12. Sensoreinheit nach Anspruch 11, dadurch gekennzeichnet, daß sich die Ansatzflächen (7) in Ausnehmungen befinden, so daß das Werkzeug betätigt werden kann, den zweiten Teil zu drehen und abzuziehen von oder einzusetzen in den ersten Teil.

13. Sensoreinheit nach einem der vorhergehenden Ansprüche, dadurch gekennzeichnet, daß der zweite Teil (3) mit wenigstens einer Schlüsselnut (7A, 7B) zum Aufnehmen eines Werkzeugs ('T') zum Drehen des zweiten Teils (3′) versehen ist.

14. Sensoreinheit nach Anspruch 13, dadurch gekennzeichnet, daß der zweite Teil (3′) zwei Schlüsselnuten (7A, 7B) aufweist, die an den gegenüberliegenden Seiten des zweiten Teils (3′) angebracht sind.

15. Sensoreinheit nach Anspruch 13 oder 14, dadurch gekennzeichnet, daß eine ringförmige Aussparung (49) zwischen dem ersten Teil (2′) und dem zweiten Teil (3′) gebildet wird, wenn der erste Teil (2′) und der zweite Teil (3′) miteinander verbunden sind.

16. Sensoreinheit nach Anspruch 14 und 15, dadurch gekennzeichnet, daß Schlüsselnuten (7A, 7B) in die ringförmige Aussparung (49) münden.

17. Sensoreinheit nach den Ansprüchen 13, 14, 15 oder 16, dadurch gekennzeichnet, daß der erste Teil (2′) mit einer ringförmigen Nut (48) zum Stützen eines Werkzeugs ('T') während des Fernaufbaus der Sensoreinheit (G′) versehen ist.

18. Werkzeug zum Abbau und Wiederaufbau einer Sensoreinheit nach Anspruch 13, dadurch gekennzeichnet, daß das Werkzeug ('T') einen Behälter (50) zum Aufnehmen des zweiten Teils (3) des Gassensors (G′) und einen ersten Hebel (56, 57) aufweist, der drehbar an dem Behälter (50) montiert ist und einen ersten Zahn (63, 65) hat, der, wenn der zweite Teil (3′) in den Behälter (50) gelangt, in die Schlüsselnut (7A, 7B) auf dem zweiten Teil (3′) greift und den zweiten Teil (3′) zur Drehung mit dem Werkzeug ('T') zwingt.

## Revendications

1. Unité de détection (G ; G′)destinée à être utilisée dans des zones de danger, ladite unité de détection comprenant une enceinte (1; 1′) comportant une première partie (2; 2′) et une seconde partie (3; 3′) montée de façon amovible sur ladite première partie (2 ; 2′), une embase (22 ; 22′) montée sur ladite première partie (2 ; 2′) et un élément de détecteur (123 ; 123′) qui est branché dans ladite embase (22 ; 22′), caractérisée en ce que ledit élément de détecteur (123 , 123′) est monté sur ladite seconde partie (3 ; 3′) de sorte que, lorsque ladite seconde partie (3 ; 3′) est séparée de ladite première partie (2 ; 2′), ledit élément de détecteur (123 ; 123′) est retiré de ladite embase (22; 22′).

2. Unité de détection selon la revendication 1, caractérisée en ce que ladite première partie (2; 2′) comprend une broche filetée (4, 4′) pour monter ladite unité de détection (G, G′) sur un boîtier de jonction (35).

3. Unité de détection selon la revendication 1 ou 2, caractérisée en ce que ladite seconde partie (3; 3′) comprend un élément interne (5; 5′) et un anneau de serrage (6; 6′) pouvant tourner par rapport audit élément interne (5; 5′)

4. Unité de détection selon la revendication 3, caractérisée en ce qu'elle comprend un circlip (11; 11′) pour empêcher la séparation dudit élément interne (5;5′) et dudit anneau de serrage (6;6′).

5. Unité de détection selon la revendication 3 ou 4, caractérisée en ce qu'elle comprend un élément de verrouillage (15; 15′) disposé entre ledit élément interne et ledit anneau de serrage (6; 6′), la rotation dudit anneau de serrage (6;6′) par rapport audit élément interne (5;5′) dans un sens étant efficace pour pousser ledit élément de verrouillage (15 ; 15′) radialement vers l'extérieur.

6. Unité de détection selon la revendication 3, 4 ou 5 , caractérisée en ce qu'elle comprend un goujon d'assemblage (18; 18′) pour aligner ladite première partie (2;2′) et ledit élément interne (5;5′) selon une orientation prédéterminée .

7. Unité de détection selon l'une quelconque des revendications précédentes, caractérisée en ce qu'elle comprend un goujon d'assemblage (34;34′) maintenant ladite embase (22;22′) dans une orientation prédéterminée par rapport à ladite première partie (2;2′).

8. Unité de détection selon l'une quelconque des revendications précédentes, caractérisée en ce que ladite embase (22;22′) est prévue avec des prises (23, 24, 25; 23′,24′,25′) pour recevoir des broches (26,27,28; 26′,27′,28′) situées sur ledit élément de détecteur (123; 123′).

9. Unité de détection selon la revendication 3, 4, 5 ou 6, la revendication 7 ou 8 lorsqu'elle dépend de la revendication 3, caractérisée en ce que ledit élément de détecteur (123,123′) est monté de façon à coulisser dans ledit élément interne (5;5′).

10. Unité de détection selon l'une quelconque des revendications précédentes, caractérisée en ce que ledit élément de détecteur (123;123′) est conçu pour détecter des gaz.

11. Unité de détection selon l'une quelconque des revendications précédentes , caractérisée en ce que ladite seconde partie (3;3′) est prévue avec des parties plates (7) en vue d'une mise en rotation à distance par l'intermédiaire d'un outil.

12. Unité de détection selon la revendication 11, caractérisée en ce que lesdites parties plates (7) reposent dans des évidements de sorte que ledit outil puisse être manipulé en vue de faire tourner ladite seconde partie et de la retirer de ou de l' insérer dans ladite première partie.

13. Unité de détection selon l'une quelconque des revendications précédentes, caractérisée en ce que ladite seconde partie (3) est prévue avec au moins une rainure de clavette (7A, 7B) destinée à recevoir un outil ("T") pour faire tourner ladite seconde partie (3′).

14. Unité de détection selon la revendication 13, caractérisée en ce que ladite seconde partie (3′) comprend deux rainures de clavette (7A, 7B), disposées sur les côtés opposés de ladite seconde partie (3′)

15. Unité de détection selon la revendication 13 ou 14 , caractérisée en ce qu'une gorge annulaire (49) est formée entre ladite première partie (2′) et ladite seconde partie (3′) lorsque ladite première partie (2′) et ladite seconde partie (3') sont connectées.

16. Unité de détection selon la revendication 14 et 15, caractérisée en ce que lesdites rainures de clavette (7A, 7B) s'ouvrent dans ladite gorge annulaire (49).

17. Unité de détection selon la revendication 13, 14, 15 ou 16, caractérisée en ce que ladite première partie (2′) est prévue avec une gorge annulaire (48) pour supporter un outil ('T') pendant un assemblage à distance de ladite unité de détection (G′).

18. Outil pour démonter et réassembler une unité de détection selon la revendication 13 , caractérisé en ce que ledit outil ('T') comprend une partie en forme de coupe (50) pour loger ladite seconde partie (3) dudit détecteur de gaz (G′) et un premier levier (56,57) qui est monté de façon à pivoter sur ladite partie en forme de coupe (50) et qui comporte une première dent (63, 65) qui , lorsque ladite seconde partie (3′) entre dans ladite partie en forme de coupe (50) pénètre dans lesdites rainures de clavette (7A, 7B) situées sur ladite seconde partie (3′) et contraint ladite seconde partie (3′) à opérer une rotation avec ledit outil ('T').
